# EUROPÄISCHE PATENTSCHRIFT

(11) **EP 0 617 623 B1**
(45) Veröffentlichungstag und Bekanntmachung des Hinweises auf die Patenterteilung: **15.05.2002**
(21) Anmeldenummer: 93900057.6
(22) Anmeldetag: 16.12.1992
(51) Int. Cl.: A61K 31/60, A61K 9/06, A61P 7/02

(54) **EIN ACETYLSALICYLSÄURE ENTHALTENDES TRANSDERMALES VERABREICHUNGSSYSTEM FÜR DIE ANTITHROMBOTISCHE THERAPIE**
TRANSDERMALLY ADMINISTERED SYSTEM CONTAINING ACETYLSALICYLIC ACID FOR THROMBOSIS THERAPY
SYSTEME D'ADMINISTRATION TRANSDERMIQUE CONTENANT DE L'ACIDE ACETYLSALICYLIQUE POUR LA THERAPIE ANTITHROMBOTIQUE

(30) Priorität: 20.12.1991 DE 4142483
(43) Veröffentlichungstag der Anmeldung: 05.10.1994
(73) Patentinhaber: LTS Lohmann Therapie-Systeme AG, 56626 Andernach (DE)
(72) Erfinder: BECHER, Frank, D-5400 Koblenz (DE); KISSEL, Thomas, D-7813 Staufen 3 (DE)
(74) Vertreter: Flaccus, Rolf-Dieter, Dr.
(86) Internationale Anmeldenummer: EP9202914
(87) Internationale Veröffentlichungsnummer: WO9312799

(56) Entgegenhaltungen:
- WO-A-92/20343
- GB-A- 2 144 326
- US-A- 3 598 122
- US-A- 4 640 689
- THE NEW ENGLAND JOURNAL OF MEDICINE, Band 321, No. 3, 1989, V. Fuster, M.D. et al., "ASPIRIN IN THE PREVENTION OF CORONARY DISEASE"

## Beschreibung

Gegenstand der Erfindung ist die Verwendung von pharmazeutisch akzeptablen Salzen der Acetylsalicylsäure zur Herstellung eines für die antithrombotische Therapie geeigneten transdermalen Systems, das die Salze der Acetylsalicylsäure in einer Matrix enthält, die die Abspaltung der Acetylgruppe von der Acetylsalicylsäure im wesentlichen unterdrückt und frei von Stoffen ist, die unter Lagerungsbedingungen bzw. während der Anwendung die Abspaltung der Acetylgruppe bewirken.

Die die Aggregation von Thrombozyten verhindernde Wirkung von Acetylsalicylsäure (ASS) und deren Wirkung bei der Vorbeugung gegen eine Herzthrombose wurde in den späten 60er Jahren beschrieben. In der Folgezeit wurde eine große Zahl klinischer Studien durchgeführt, bel denen ASS oral bei den folgenden Indikationen eingesetzt wurde:
Verhinderung eines erstmaligen Herzinfarktes
Verhinderung eines Reinfarktes
Behandlung von instabiler Angina Pectoris
Thromboseprophylaxe nach Einsatz von Gefäßprothesen bzw. künstlichen Herzklappen
Thromboseprophylaxe der peripheren arteriellen Gefäße
Thromboseprophylaxe cerebraler Mangeldurchblutung

Wenn im folgenden der Ausdruck "antithrombotische Therapie" benutzt wird, sind im wesentlichen diese Indikationen umfaßt.
Die Ergebnisse dieser therapeutischen Studien an Patienten sind in letzter Zeit zusammengefaßt worden (V. Fuster et al., "Aspirin in the prevention of coronary disease", New Engl. J. Med. 321, 183-185 (1989) und R. Zichner et al., "Zur optimalen Dosierung von Acetylsalicylsaeure", Med. Klin. 84, 43-51 (1989)).

Acetylsalicylsäure wird in der medizinischen Praxis häufig als nichtsteroidaler entzündungshemmender, analgetischer und antipyretischer Wirkstoff eingesetzt. ASS beeinflußt die Thrombozytenfunktion und verhindert eine Thrombose durch eine irreversible Hemmung der Thromboxan A2 Synthese (M. Buchanan et al., "Aspirin inhibits platelet function independent of cyclooxygenase", Thrombosis Res. 25, 363-373 (1982)).

Nach oraler Gabe wird ASS schnell absorbiert. Die biologische Halbwertzeit im Körperkreislauf ist jedoch sehr kurz, sie dauert nur 15-20 Minuten (M. Rowland et al., "Kinetics of acetylsolicylic acid disposition in man", Nature 215, 413-414 (1967)). Bei normalen Erwachsenen wird ASS schnell schon im Magendarmtrakt zu Salicylsäure hydrolysiert (G. Levy, "Clinical pharmacokinetics of aspirin", Pediatrics 62, 867-872 (1978)).

Es sollte jedoch festgehalten werden, daß ASS selbst und nicht dos Hydrolyseprodukt Salicylsäure bei der Hemmung der Thrombozytenfunktion aktiv ist (W. Horsch. "Die Salicylate", Pharmazie 34, 585-604 (1979)).

Acetylsalicylsäure (ASS) wird insbesondere in den USA von weiten Bevölkerungskreisen laufend eingenommen. Nach einer Arbeit von Thun et al., "Aspirin Use and Reduced Risk of Fatal Colon Cancer", New Engl. J. Med. 325, 1593-1596 (1991) verringert ASS die durch Dickdarmkrebs verursachte Mortalität auf etwa die Hälfte, wenn ASS laufend, und zwar an mindestens 16 Tagen monatlich, eingenommen wurde. Die Untersuchung erstreckte sich auf mehr als 660.000 Personen, die ASS mindestens ein Jahr lang eingenommen haben und in allen 50 Staaten der USA, im Distrikt von Columbia und in Puerto Rico lebten. Wenngleich nur auf den Gebrauch (use) von ASS Bezug genommen wurde, ohne daß weitere Angaben über die Art der Verabreichung und die Dosis gemacht wurden, so ist doch davon auszugehen, daß die ASS oral genommen worden war und daß die für die Wirkung verantwortliche Substanz nicht das Hydrolyseprodukt Salicylsäure ist, sondern ASS an sich.

Bei der antithrombotischen Therapie wird fast ausschließlich die orale Verabreichung praktiziert; dagegen sind bei den entzündungshemmenden, analgetischen und ontipyretischen Indikationen schon Versuche bekannt geworden, den Wirkstoff auch über die Haut zu applizieren. So wird ASS in US-A-3,598,122 als möglicher antipyretischer Wirkstoff in einem membrongesteuerten transdermalen therapeutischen System erwähnt. Die FR-M 1757 beschreibt die dermale topische Anwendung einer Öl-in-Wasser-Emulsion. die 5% ASS gegen akuten Schmerz enthölt. In der FR-A 2 297 612 werden Einreibemittel und Salben beansprucht, die ASS als analgetisches Mittel enthalten. Mit Corticosteroiden kombiniert wird ASS in US-A-4,012,508 für die topische Anwendung bei dermatologischen Indikationen eingesetzt. Die US-A-4,219,548 beschreibt eine topische Anwendung von ASS zur Entzündungshemmung. Ein ASS-haltiges Gel wird in der EP-A 0 055 635 bei entzündungshemmenden, analgetischen und antipyretischen Indikationen topisch appliziert. Eine Vorrichtung zur transdermalen Applikation von ASS aus wäßrigem System zur Erzielung entzündungshemmender und analgetischer Effekte ist Gegenstand der US-A-4,460,368 . Aus ethonolischer Lösung wird ASS in der US-A-4,665,063 gegen dermatologische Störungen topisch appliziert.

Eine Steigerung der Penetrationsrate der ASS bei transdermaler Applikation wird in der US-A-4,640,689 mit elektrischem Strom erreicht.
Auch der Zusatz von geeigneten Penetrationsbeschleunigern gemäß EP-A 162 239 führt bei einer transdermalen Anwendung zu einer verbesserten Penetration der ASS durch die Haut. Nach der JP-A-61 167 615 wird ASS mit Hilfe eines Filmes auf der Haut appliziert. Die US-A-4,810,699 beschreibt Kombinationen von ASS mit anderen Wirkstoffen zur transdermalen Behandlung von Entzündungen, Schmerzen und Fieber. Spezielle Penetrationsbeschleuniger für die transdermale Applikation von ASS als Schmerzmittel sind Inhalt der JP-A-1 203 336, Weitere Substanzen dieser Art für ASS bei transdermaler Applikation zur Entzündungshemmung finden sich in der JP-A-1,242,521. Lagerstabile Lösungen von ASS zur toplschen Applikation mit dem Ziel der Entzündungshemmung und Schmerzlinderung sind schließlich Gegenstand der US-A-4,975,269.

Dem geschilderten Stand der Technik ist nicht zu entnehmen und es ist auch nicht daraus herzuleiten, daß die Verwendung eines transdermalen Systems in Betracht gezogen wird, das pharmazeutisch akzeptable Salze der ASS zur Verhinderung der Thrombozytenaggregation beim Menschen enthält.

Viele Formulierungen und Zusammensetzungen enthalten Wasser oder hydrophile Lösemittel, die die Hydrolyse von ASS zur Salicylsäure beschleunigen. Da diese, wie weiter oben schon dargelegt, keine antithrombotische Wirkung entfaltet, dagegen aber mit der ASS vergleichbare entzündungshemmende und analgetische Wirkung zeigt, wird verständlich, daß der Abbau von ASS in den erwähnten Applikationssystemen nicht im Detail untersucht worden ist. So wurde auch in der nicht vorveröffentlichten WO-A-92/20343 das Problem der Hydrolyse von ASS zu Salicylsäure weder behandelt noch andeutungsweise erwähnt. Vielmehr werden für die bekannte Formulierung bei Verabreichung über die Haut Zusätze von Propylenglykol und Alkoholen, ausgesucht aus der Gruppe von Isopropylalkohol und Ethylalkohol empfohlen.

Es war deshalb die Aufgabe der vorliegenden Erfindung, für die Applikation von ASS und/oder ihrer pharmazeutisch akzeptablen Salze zur antithrombotischen Therapie ein Verabreichungssystem bereitzustellen, das die Nachteile der oralen Applikation vermeidet und eine gezielte Dosierung des unveränderten Wirkstoffes erlaubt.

Die Lösung dieser Aufgabe besteht nun überraschenderweise darin, daß pharmazeutisch akzeptable Salze der Acetylsalicylsäure zur Herstellung eines für die antithrombotische Therapie geeigneten transdermalen Systems (TTS) verwendet werden, wobei das TTS die Salze der Acetylsalicylsäure in einer Matrix enthält, die die Abspaltung der Acetylgruppe von der Acetylsalicylsäure im wesentlichen unterdrückt und frei von Stoffen ist, die unter Lagerungsbedingungen bzw. während der Anwendung die Abspaltung der Acetylgruppe bewirkt.

Eine weitere erfindungsgemäße Lösung besteht darin, daß Acetylsalicylsäure zur Herstellung eines für die antithrombotische Therapie geeigneten transdermalen Systems, das die Acetylsalicylsäure und/oder deren pharmazeutisch akzeptablen Salze in einer Matrix enthält, verwendet wird, wobei die Matrix die Abspaltung der Acetylgruppe von der Acetylsalicylsäure im wesentlichen unterdrückt und frei von Stoffen ist, die unter Lagerungsbedingungen bzw. während der Anwendung die Abspaltung der Acetylgruppe bewirken, und wobei die Matrix zur Zurückdrängung der Hydrolyse ein Acylierungsmittel, vorzugsweise Acetylierungsmittel und insbesondere Acetanhydrid enthält.

Ein transdermales Verabreichungssystem bietet bei der antithrombotischen Therapie folgende Vorteile:
1. ASS wird in ihrer pharmakologisch aktiven Form direkt in den Körperkreislauf gegeben, wodurch ein Stoffwechsel im gastrointestinalen Trakt vermieden wird.
2. Reduzierung von gastrointestinalen Nebenwirkungen
3. Konstante therapeutische Wirkung mit verringerten ASS-Gaben.
4. Vermindertes Risiko einer Überdosierung.
5. Ambulante Behandlung von Patienten ohne den Zwang einer Über wachung.
6. Verbesserte Patiententherapietreue

Der Gehalt an ASS in einer derartigen Verabreichungseinheit liegt im allgemeinen bei 5 bis 500, vorzugsweise 30 bis 200 mg bzw. der entsprechenden Menge eines pharmazeutisch akzeptablen Salzes. Die hier verwendbaren ASS-Satze sind alle nicht toxischen, pharmakologisch wirksamen Salze wie die Lithium-. Natrium-, Kalium-, Magnesium- und Kalziumsalze oder Salze von ASS mit basischen organischen Verbindungen wie Lysin, Arginin oder Cetrimoniumbromid (Hexadecyltrimethylammoniumbromid). Die Geschwindigkeit und das Ausmaß der transdermalen Überführung der ASS in den Körper ist naturgemäß von der Menge, der Art der Verbindung (freie Säure oder Salz) und gegebenenfalls auch von der Gegenwort von Hilfsstoffen wie Penetrationsbeschleunigern abhängig. Zweckmäßig stellt man das System so ein, daß sich ein ASS-Blutspiegelwert zwischen 0,1 und 1,0 µg/ml einstellt. Für die praktische Anwendung wird der Gehalt zweckmäßig auf die Art der Matrix, die empfohlene Tragedauer des Pflasters, die beabsichtigte Indikation, das Körpergewicht (Kinder bzw. Erwachsene), die Permeabilität der Matrix bzw. Membran des Pflasters und die Permeation durch die Haut abgestimmt.
Eine bei der antithrombotischen Therapie therapeutisch wirksame Menge von ASS und/oder ASS-Satzen im Blut entspricht ASS-Blutspiegelwerten zwischen 0.1 und 1.0 µg/ml. Obwohl ASS noch oraler Gabe schnell absorbiert wird, ist wegen der Hydrolyse von ASS zu Salicylsäure diese Art der Verabreichung ungünstig, besonders wenn die kurze biologische Halbwertzeit und die Tatsache berücksichtigt wird, daß für eine Prophylaxe eine möglichst konstante Verabreichung erwünscht ist. Durch die erfindungsgemöß vorgeschlagene transdermale Behandlung erhält man dagegen ziemlich konstante und reproduzierbare ASS-Blutspiegelwerte, die bei der antithrombotischen Therapie besonders wirksam sind. Ein erfindungsgemäßes transdermales Abgabesystem stellt dagegen konstante und reproduzierbare ASA-Blutspiegel sicher, die bei der antithrombotischen Therapie wirksam sind.

Das erfindungsgemäß verwendete transdermale Verabreichungssystem für ASS und/oder ASS-Salze kann in vielfältiger Weise realisiert werden, so z.B. als insbesondere haftklebendes Pflaster, als Film, als Spray, Creme, Salbe und ähnliches. Bevorzugt ist die Verabreichungsform des haftklebenden Pflasters, welches eine undurchlässige Rückschicht, ein damit verbundenes Wirkstoffreservoir aus einer Polymermatrix. bei Abwesenheit anderer Steuermechanismen eine die Abgabe des Wirkstoffs steuernde Membran, eine Haftklebeeinrichtung zur Befestigung des Systems auf der Haut und im Bedarfsfall eine vor der Applikation des Systems wieder ablösbore Schutzschicht umfasst. Bei allen Formen ist darauf zu achten, daß die das Wirkstoffreservoir bildende Matrix so gewählt ist, daß die Hydrolyse von ASS unterbleibt oder zumindest stark zurückgedrängt ist. Eine hydrophobe Einstellung der Matrix führt hier eher zum Ziele als eine hydrophile.
Zur Zurückdrängung bzw. Unterdrückung der Hydrolyse kann man Stoffe zusetzen wie Acylierungsmittel, vorzugsweise Acetylierungsmittel, insbesondere Acetanhydrid, z.B. in einer Menge von 0,01 bis 3, vorzugsweise von 0,1 bis 2 Gew.-%. bezogen auf Acetylsalicylsäure.

Die erfindungsgemöß brauchbaren transdermalen haftklebenden Pflaster sind alle Pflaster, die dem Fachmann aus dem Stand der Technik bekannt sind. Sie lassen sich weitgehend zwei grundsätzlichen Steuerungsprinzipien zuordnen: Matrix-Diffusions-Steuerung und Membran-Steuerung, wobei nur die letztere eine Wirkstofffreisetzung nullter Ordnung besitzt. Ein Pflaster mit Matrix-Diffusions-Steuerung wird z.B. in DE-A-33 15 272 beschrieben. Es besteht aus einer undurchlässigen Rückschicht, einem damit verbundenen Reservoir aus einer Polymermatrix, das den Wirkstoff in einer Konzentration oberhalb der Söttigungskonzentration enthält, einer mit dem Reservoir verbundenen, für den Wirkstoff durchlässigen Haftklebeschicht und einer die Haftklebeschicht abdeckenden, zum Gebrauch wieder ablösbaren Schutzschicht, z.B. einer silikonisierten Folie aus Polyester, insbesondere Polyethylenterephthalat. Ist die Reservoirmatrix selbst schon haftklebend, so kann auf die zusätzliche Hattklebeschicht verzichtet werden. Es sind aber auch Systeme mit geringerer als der Sättigungskonzentration möglich.
Für Pflaster mit Membran-Steuerung sei beispielhaft auf die US-Patentschriften 3,742,951, 3,797,494, 3,996,934 und 3,031,894 hingewiesen. Diese Pflaster bestehen grundsätzlich aus einer Rückschicht (z.B. einer Folie aus einem Polyester wie Polyethylenterephtalat, die oluminisiert sein kann, oder einer aluminisierten Folie aus einem Kunstharz, wie Polypropylen, Nylon, Polycaprolactam), die eine der Oberflächen darstellt, einer Membran, einer für den Wirkstoff durchlässigen Klebeschicht, die die andere Oberfläche darstellt und letztlich einem Reservoir, das den Wirkstoff zwischen den beiden die Oberflächen bildenden Schichten enthält. Alternativ dazu kann der Wirkstoff auch in einer Vielzahl von Mikrokapseln enthalten sein, die innerhalb der durchlässigen Klebschicht verteilt sind. In jedem Fall wird der Wirkstoff aus dem Reservoir oder den Mikrokapseln durch eine Membran in die für den Wirkstoff durchlässige Klebschicht, die im Kontakt mit der Haut des zu Behandelnden steht, kontinuierlich abgegeben. Im Falle von Mikrokapseln kann das Kapselmaterial auch als Membran wirken. Für Membranen und Mikrokapseln geeignete Stoffe sind z.B. in der US-A-3,996,934 beschrieben.

Ergänzend sei noch darauf hingewiesen, doß auch eine Steuerung mit Hilfe von elektrischem Strom möglich ist, wobei der Durchtritt des Wirkstoffs durch die Haut den geschwindigkeitsbestimmenden Schritt darstellt. Derartige Vorgänge werden mit Elektroosmose, lontophorese oder Elektrophorese bezeichnet.

Die Pflaster, gleich welcher Art, können im Bedarfsfall neben der das Reservoir bildenden Matrix und dem Wirkstoff, wozu auch Kombinationen von ASS und deren Salzen zählen, noch verschiedenartige Zusatzstoffe enthalten, um das gewünschte Eigenschaftsbild zu erhalten. Besonders erwähnt werden sollen solche Zusatzstoffe, die die Permeation von ASS und/oder deren pharmazeutisch akzeptablen Salzen durch die Haut fördem. Eine genaue Aufzählung der Zusatzstoffe erübrigt sich für den Fachmann auf diesem Gebiet. jedoch seien beispielsweise genannt Glycerin, 1,2-Propandiol, der Monomethyl- bzw. Monoethylether des Ethylenglykols, 2-Octyldodecanol, das Laurat, Palmitat, Stearat oder Oleat des Sorbits, C₈-C₁₀-ethoxylierte Ölsäureglyceride, niedere Alkyl(C₁ bis C₃)-Ester der Laurinsäure, wie Propylenglykolmonolaurat, Laurin-, Caprin-, Ölsäure usw. Die Menge betrögt im allgemeinen von 0 bis 20, vorzugsweise von 0,5 bis 10 Gew.-%, bezogen auf die Gesamtbestandteile der Matrix. Sie ist abhängig von der Art der Matrix, der Permeabilität der Matrix bzw. Membran des Pflasters, dem Lösungsvermögen des Penetrationsbeschleunigers für den Wirkstoff und der Permeation durch die Haut.

Die Erfindung wird durch folgende Beispiele erläutert:

### Beispiele:

### 1. Einschichtiges System auf Acrylatbasis.

Zu 100 g Lösung eines Acrylatklebers (z.B. Durotak ^{R} 280-2516 National Starch and Chemical,) mit einem Feststoffgehalt von 42 Gew.-% werden 5 g Dioctylcyclohexan, 8 g Acetylsalicylsäure und 40 mg Acetanhydrid gegeben und die Lösung durch Rühren homogenisiert.

Die Lösung wird dann 300 µm dick auf eine silikonisierte, 100 µm dicke Polyesterfolie gestrichen. Diese Folie übernimmt beim fertigen System die Funktion der wiederentfembaren Schutzschicht und muß vor Gebrauch entfernt werden. Der feuchte Film wird 20 Minuten bei 50°C getrocknet und hat danach ein Flächengewicht von 100 g/m².

Anschließend wird der getrocknete Film mit einer 12 µm dicken Polyesterfolie kaschiert. Aus dem Laminat werden die fertigen Pflaster gestanzt.

### 2. Mehrschichtiges System

Das fertige System besteht aus einer wiederentfernbaren Schutzschicht, einem Hautkleberstrich, einem nichtklebenden Reservoir, einer wirkstoffundurchlässigen Rückschicht und einem gut klebenden Grundierstrich, der sich zwischen Reservoirschicht und Rückschicht befindet und die Aufgabe hat, das nichtklebende Reservoir auf der Rückschicht zu verankern.

### A. Herstellung des Hautkleberstriches

100 g eines Blockpolymers aus Polystyrol und Polyisopren (z.B. Cariflex ^{R}TR-1107, Fa. Shell),
175 g eines Glycerinesters von partiell hydriertem Kolophonium
   und
50 g Dioctylcyclohexan
werden in 500 g n-Heptan gelöst und anschließend 15 g Acetylsalicylsäure und 150 mg Acetanhydrid zugegeben. Die Masse wird durch Rühren homogenisiert und danach 100 µm dick auf eine beim fertigen Produkt als wiederentfernbare Schutzschicht dienende silikonisierte Polyesterfolie gestrichen. Der feuchte Film wird 20 Minuten bei 50°C getrocknet und hat dann ein Flächengewicht von 25 g/m².

### B. Herstellung des Reservoirstriches

100 g eines Blockpolymers aus Polystyrol und Polyisopren (z.B. Cariflex TR-1107, Fa. Shell)
   und
20 g Dioctylcyclohexan werden in 120 g n-Heptan gelöst.

Anschließend werden 40 g Acetylsalicylsäure und 40 mg Acetanhydrid zugegeben und die Masse durch Rühren homogenisiert. Die Masse wird 300 µm dick auf eine stärker als die wiederentfembare Schutzschicht silikonisierte Polyesterfolie gestrichen und 20 Minuten bei 50°C getrocknet. Der getrocknete Reservoirfilm hat ein Flächengewicht von 100 g/m².

### C. Herstellung des Grundierstriches

100 g eines Blockpolymers aus Polystyrol und Polyisopren (z.B. Coriflex TR-1107. Fa. Shell),
175 g eines Glycerinesters von partiell hydriertem Kolophonium
   und
50 g Dioctylcyclohexan
werden in 500 g n-Heptan gelöst und 100 µm dick analog zu B auf eine stärker als die wiederentfernbare Schutzschicht silikonisierte Polyesterfolie gestrichen und 20 Minuten bei 50°C getrocknet. Der getrocknete Film hat ein Flächengewicht von 25 g/m².

### D. Aufbau des Gesamtsystems und Stanzen der Einzelpflaster

Der unter B erhaltene Reservoirstrich wird auf den Hautkleberstrich A aufkaschiert und anschließend die unter B genannte stärker silikonisierte Folie entfernt. Nun wird in der gleichen Weise der Grundierstrich C aufgebracht und nach dem Entfernen der unter C genannten stärker silikonisierten Folie eine 12 µm dicke Polyesterfolie aufkaschiert.

Die fertigen Pflaster werden aus dem Gesamtlaminat ausgestanzt.

### 3. Membransystem

Ein heißsiegefähiges Laminat aus einer flexiblen Polyesterfolie und einer Folie aus einem Polyethylen/Vinylacetat-Copolymer wird entsprechend den Ausmaßen und Formen der späteren Pflaster so gegen eine 50 µm dicke Membran aus einem Polyethylen/Vinylacetot-Copolymer mit einem Vinylacetatgehalt von 19% gesiegelt, daß eine Art flacher Beutel entsteht. Die Siegelnaht soll eine Breite von 4 mm haben. Bevor der Beutel lückenlos versiegelt ist, wird er mit einer flüssigen Zubereitung aus einem Silikonöl mit 10% Acetylsalicylsäure und 0,05% Acetanhydrid befüllt.

Die Membranseite der Beutel wird nun auf einen sich auf einer geeigneten, abhäsiv ausgerüsteten Folie befindlichen Hautkleberstrich auf Silikonbasis kaschiert. Diese Folie ist identisch mit der wiederentfembaren Schutzschicht.

Die fertigen Systeme werden so ausgestanzt, daß ein Beutel mit einem 3 mm starken Siegelrand verbleibt.

## Patentansprüche

1. Verwendung von pharmazeutisch akzeptablen Salzen der Acetysalicylsäure zur Herstellung eines für die antithrombotische Therapie geeigneten transdermalen Systems, das die Salze der Acetysalicylsäure in einer Matrix enthält, die die Abspaltung der Acetylgruppe von der Acetylsalicylsäure im wesentlichen unterdrückt und frei von Stoffen ist, die unter Lagerungsbedingungen bzw. während der Anwendung die Abspaltung der Acetylgruppe bewirken.

2. Verwendung nach Anspruch 1, **dadurch gekennzeichnet, daß** es als Wirkstoff Acetylsalicylsäure in Form ihrer pharmazeutisch akzeptablen Salze und zusätzlich freie Acetylsalicylsäure enthält.

3. Verwendung nach Anspruch 1 oder 2, **dadurch gekennzeichnet, daß** das System zwischen 5 und 500 mg, vorzugsweise zwischen 30 und 200 mg, eines pharmazeutisch akzeptablen Salzes der Acetylsalicylsäure in stabiler Form enthält, wobei die Mengenangabe auf freie Acetylsalicylsäure berechnet ist.

4. Verwendung nach einem oder mehreren der Ansprüche 1-3, **dadurch gekennzeichnet, daß** das System als Creme oder Salbe vorliegt.

5. Verwendung nach einem oder mehreren der Ansprüche 1-4, **dadurch gekennzeichnet, daß** das System hergestellt wurde, indem eine pharmazeutisch wirksame Menge von pharmazeutisch akzeptablen Salzen der Acetylsalicylsäure und gegebenenfalls freier Acetysalicylsäure in fester Form oder in Lösung oder in Dispersion in das Verabreichungssystem eingebracht wurde, gegebenenfalls unter Zugabe üblicher Zusatzstoffe.

6. Verwendung von Acetylsalicylsäure zur Herstellung eines für die antithrombotische Therapie geeigneten transdermalen Systems, das die Acetylsalicylsäure und/oder deren pharmazeutisch akzeptablen Salze in einer Matrix enthält, die die Abspaltung der Acetylgruppe von der Acetylsalicylsäure im wesentlichen unterdrückt und frei von Stoffen ist, die unter Lagerungsbedingungen beziehungsweise während der Anwendung die Abspaltung bewirken und wobei die Matrix zur Zurückdrängung der Hydrolyse ein Acylierungsmittel, vorzugsweise Acetylierungsmittel und insbesondere Acetanhydrid enthält.

7. Verwendung nach Anspruch 6, **dadurch gekennzeichnet, daß** das Acetanhydrid in einer Menge von 0,01-3, vorzugsweise von 0,1-2 Gew.-% bezogen auf den Acetylsalicylsäuregehalt, zugegen ist.

8. Verwendung nach Anspruch 6 oder 7, **dadurch gekennzeichnet, daß** das System hergestellt wurde, indem eine pharmazeutisch wirksame Menge von Acetylsalicylsäure und/oder deren pharmazeutisch akzeptablen Salzen in fester Form oder in Lösung oder in Dispersion in das Verabreichungssystem eingebracht wurde, gegebenenfalls unter Zugabe üblicher Zusatzstoffe.

9. Verwendung nach einem oder mehreren der Ansprüche 1-3 und 5-8, **dadurch gekennzeichnet, daß** das System eine undurchlässige Rückschicht, ein damit verbundenes wirkstoffhaltiges Reservoir aus einer Polymermatrix, vorzugsweise mit einer Wirkstoffkonzentration oberhalb der Sättigungskonzentration, bei Abwesenheit anderer Steuermechanismen eine die Abgabe des Wirkstoffs steuernde Membran, eine Haftklebeeinrichtung zur Befestigung des Systems auf der Haut und im Bedarfsfall eine vor der Applikation des Systems wieder ablösbare Schutzschicht umfaßt.

10. Verwendung nach einem oder mehreren der Ansprüche 1-9, **gekennzeichnet durch** die Einbeziehung von die Permeation von Acetylsalicylsäure und/oder deren pharmazeutisch akzeptablen Salzen **durch** die Haut fördernden Mitteln in Form von geeigneten Zusatzstoffen und/oder elektrischem Strom.

11. Verwendung gemäß einem oder mehreren der Ansprüche 1-10, **dadurch gekennzeichnet, daß** das System für die Humanmedizin bestimmt ist und insbesondere in Form eines haftklebenden Pflasters vorliegt.

## Claims

1. The use of pharmaceutically acceptable salts of acetylsalicylic acid for the production of a transdermal system suitable for antithrombotic therapy, which system contains the salts of acetylsalicylic acid in a matrix which substantially suppresses the separation of the acetyl group from the acetylsalicylic acid and which is free of substances which, under storage conditions or when the system is being used, effect the separation of the acetyl group.

2. The use according to claim 1, **characterized in that** the system contains as active substance acetylsalicylic acid in the form of its pharmaceutically acceptable salts, and, in addition, free acetylsalicylic acid.

3. The use according to claim 1 or 2, **characterized in that** the system contains between 5 and 500 mg, preferably between 30 and 200 mg, of a pharmaceutically acceptable salt of acetylsalicylic acid in a stable form, this indication of the amount being calculated on the free acetylsalicylic acid.

4. The use according to one or more of claims 1-3, **characterized in that** the system is present as a cream or ointment.

5. The use according to one or more of claims 1-4, **characterized in that** the system was produced by introducing, in solid form or in solution or in dispersion, a pharmaceutically effective amount of pharmaceutically acceptable salts of acetylsalicylic acid and, optionally, of free acetylsalicylic acid into the administration system, optionally with addition of common additives.

6. The use of acetylsalicylic acid for the production of a transdermal system suitable for antithrombotic therapy, which system contains the acetylsalicylic acid and/or pharmaceutically acceptable salts thereof in a matrix which substantially suppresses the separation of the acetyl group from the acetylsalicylic acid and which is free of substances which, under storage conditions or when the system is being used, effect the separation, and the said matrix containing, for suppression of hydrolysis, an acylating agent, preferably an acetylating agent and, in particular, acetic anhydride,

7. The use according to claim 6, **characterized in that** the acetic anhydride is added in an amount of 0.01-3, preferably 0.1-2%-wt, relative to the content of acetylsalicylic acid.

8. The use according to claim 6 or 7, **characterized in that** the system was produced by introducing, in solid form or in solution or in dispersion, a pharmaceutically effective amount of acetylsalicylic acid and/or of pharmaceutically acceptable salts thereof into the administration system, optionally with addition of common additives.

9. The use of one or more of claims 1-3 and 5-8, **characterized in that** the system comprises an impermeable backing layer, an active substance-containing reservoir connected thereto and made of a polymer matrix, preferably having an active substance concentration which is above the saturation concentration, where other control mechanisms are not present a membrane controlling the release of the active substance, a pressure-sensitive adhesive device for fixing the system to the skin, and, if required, a protective layer detachable prior to application.

10. The use according to one or more of claims 1-9, **characterized by** employing means in the form of suitable additives and/or electric current, which means enhance the permeation of acetylsalicylic acid and/or of the pharmaceutically acceptable salts thereof through the skin.

11. The use according to one or more of claims 1-10, **characterized in that** the system is intended for human medicine and, in particular, is present in the form of a pressure-sensitive adhesive plaster.

## Revendications

1. Utilisation de sels pharmaceutiquement acceptables de l'acide acétylsalicylique pour la fabrication d'un système transdermique approprié pour la thérapie antithrombotique qui contient les sels de l'acide acétylsalicylique dans une matrice qui empêche essentiellement la séparation du groupe acétyle de l'acide acétylsalicylique et qui est exempte de substances qui, dans des conditions d'entreposage respectivement au cours de l'application, déclenchent la séparation du groupe acétyle.

2. Utilisation selon la revendication 1, **caractérisée en ce qu'**elle contient, à titre de substance active, de l'acide acétylsalicylique sous la forme de ses sels pharmaceutiquement acceptables et en outre de l'acide acétylsalicylique libre.

3. Utilisation selon la revendication 1 ou 2, **caractérisée en ce que** le système contient un sel pharmaceutiquement acceptable de l'acide acétylsalicylique sous forme stable, en une quantité entre 5 et 500 mg, de préférence entre 30 et 200 mg, la quantité étant calculée par rapport à l'acide acétylsalicylique libre.

4. Utilisation selon une ou plusieurs des revendications 1 à 3, **caractérisée en ce que** le système est présent sous forme d'une crème ou d'un onguent.

5. Utilisation selon une ou plusieurs des revendications 1 à 4, **caractérisée en ce qu'**on prépare le système en introduisant dans le système d'administration, une quantité pharmaceutiquement efficace de sels pharmaceutiquement acceptables de l'acide acétylsalicylique et, le cas échéant, de l'acide acétylsalicylique libre, sous forme solide ou en solution ou en dispersion, le cas échéant, en ajoutant des additifs habituels.

6. Utilisation d'acide acétylsalicylique pour la préparation d'un système transdermique approprié pour la thérapie antithrombotique qui contient l'acide acétylsalicylique et/ou ses sels pharmaceutiquement acceptables dans une matrice qui empêche essentiellement la séparation du groupe acétyle de l'acide acétylsalicylique et qui est exempte de substances qui, dans des conditions d'entreposage respectivement au cours de l'application, déclenchent la séparation du groupe acétyle, et dans laquelle la matrice, dans le but de repousser l'hydrolyse, contient un agent d'acylation, de préférence un agent d'acétylation et en particulier, de l'acétanhydride,

7. Utilisation selon la revendication 6, **caractérisée en ce qu'**on ajoute l'acétanhydride en une quantité de 0,01 à 3, de préférence de 0,1 à 2 % en poids rapportés à la teneur en acide acétylsalicylique.

8. Utilisation selon la revendication 6 ou 7, **caractérisée en ce qu'**on prépare le système en introduisant dans le système d'administration, une quantité pharmaceutiquement efficace de sels pharmaceutiquement acceptables de l'acide acétylsalicylique et, le cas échéant, de l'acide acétylsalicylique libre, sous forme solide ou en solution ou en dispersion, le cas échéant, en ajoutant des additifs habituels.

9. Utilisation selon une ou plusieurs des revendications 1 à 3 et 5 à 8, **caractérisée en ce que** le système comprend une couche dorsale imperméable, un réservoir contenant la substance active relié à la première citée, constitué d'une matrice polymère, de préférence avec une concentration de substance active supérieure à la concentration de saturation, en l'absence d'autres mécanismes de réglage, une membrane réglant la libération de la substance active, un mécanisme d'auto-adhérence pour la fixation du système sur la peau et, en cas de nécessité, une couche de protection qui peut être retirée avant l'application du système.

10. Utilisation selon une ou plusieurs des revendications 1 à 9, **caractérisée par** l'incorporation d'agents favorisant la perméation de l'acide acétylsalicylique et/ou de ses sels pharmaceutiquement acceptables à travers la peau, sous la forme d'additifs appropriés et/ou de courant électrique.

11. Utilisation selon une ou plusieurs des revendications 1 à 10, **caractérisée en ce que** le système est destiné à la médecine humaine et est présent en particulier sous la forme d'un emplâtre auto-adhésif.
